# EUROPEAN PATENT SPECIFICATION

(11) **EP 0 854 731 B1**
(45) Date of publication and mention of the grant of the patent: **22.01.2003**
(21) Application number: 96932066.2
(22) Date of filing: 21.09.1996
(51) Int. Cl.: A61K 47/34, A61K 9/51, A61K 9/127

(54) **COPOLYMERIC MICELLE DRUG COMPOSITION AND METHOD FOR THE PREPARATION THEREOF**
ARZNEIMITTEL AUF DER BASIS COPOLYMERER MIZELLEN UND VERFAHREN ZU DEREN HERSTELLUNG
COMPOSITION DE MEDICAMENT A MICELLE COPOLYMERE ET SON PROCEDE DE PREPARATION

(30) Priority: 21.09.1995 KR 9530981
(43) Date of publication of application: 29.07.1998
(73) Proprietor: Sam Yang Co., Ltd., Seoul 110-470 (KR)
(72) Inventor: KIM, Sung, Chul, Seo-gu, Daejeon 302-150 (KR); CHANG, Eun, Ok, Daeduk-gu, Daejeon 306-190 (KR); SONG, In, Suk, Yuseong-gu, Daejeon 305-348 (KR); PAI, Chaul, Min, Yuseong-gu, Daejeon 305-333 (KR)
(74) Representative: Grünecker, Kinkeldey, Stockmair & Schwanhäusser Anwaltssozietät
(86) International application number: PCT/KR96/00163
(87) International publication number: WO 97/010849

(56) References cited:
- EP-A- 0 092 918
- EP-A- 0 166 596
- EP-A- 0 552 802
- WO-A-95/03357
- US-A- 5 384 333

## Description

The present invention relates to a drug delivery system, and more particularly, to a polymeric micelle drug composition comprising a hydrophobic drug and a block copolymer having a hydrophilic polymer component and a hydrophobic biodegradable polymer component.

Many important drugs are hydrophobic and have limited solubilities in water. In order to attain the expected therapeutic effect of such drug, it is usually required that a solubilized form of the drug is administered to a patient. For this purpose, there have been developed a number of methods, which are based on the use of: auxiliary solvents; surfactants; soluble forms of the drug, e.g., salts and solvates; chemically modified forms of the drug, e.g., prodrugs; soluble polymer-drug complexes; special drug carriers such as liposome; and others. However, because each of the above methods is hampered by one or more particular problems, e.g., the method based on the use of surfactant micelles to solubilize hydrophobic drugs has problems in that most of the surfactants are relatively toxic and that precipitation of the surfactant occurs when subjected to dilution.

To solve above-mentioned problems associated with surfactants, EP No. 0 552 802 A2 discloses a method for preparing micelle-shaped polymer particles by chemically fixing micelles having poly(ethylene oxide) as the hydrophilic component and a biodegradable polymer block which can be crosslinked in an aqueous phase as the hydrophobic component. That is, chemically fixed polymer particles are prepared by chemically crosslinking the hydrophobic component that constitutes the core, so as to mimic stabilized polymeric micelles. However, a crosslinking agent, or other means such as UV and heating with or without added peroxides, must be used in order to introduce crosslinking to the hydrophobic component of the block copolymer. Moreover, the biocompatibility or the safety of such crosslinked polymer particles has not yet been established.

There have been reported other studies on biodegradable block copolymer micelles having surfactant-like properties, and particularly noteworthy are the attempts to incorporate hydrophobic drugs into block copolymer micelles stabilized due to the specific nature and properties of the copolymer.

For example, EP No. 0 397 307 A2 discloses micelles of an AB type diblock copolymer which contains poly(ethylene oxide) as the hydrophilic component and poly(amino acid), e.g., polyaspartic acid, polyglutamic acid and polylysine, as the hydrophobic component, wherein therapeutically active agents are chemically bonded to the hydrophobic component of the polymer. However, it is difficult to prepare a polymer bearing specified functional groups, and there also exists the problem that such composition having a chemically bonded drug may not be safe for human use.

EP No. 0 583 955 A2 discloses a method for physically incorporating hydrophobic drugs into diblock copolymer micelles described in EP No. 0 397 307 A2. This method, thus, solves the potential safety problem arising from chemically bonding drugs to micelles. However, the poly(amino acid) segment may induce an immunoreaction and the use of an organic solvent in the preparation of the formulation may pose a problem. Further, because the peptide bonds are cleaved by enzymes in the body, it is difficult to control the release rate of the drug incorporated therein.

US-A-5 384 333 discloses a bio-injectable drug composition comprising a solid polymer matrix containing the active agent.

WO-A-95-03357 discloses particles comprising a biodegradable core which is solid, containing a biologically active agent.

The present inventors therefore have studied the various prior art materials and have provided the present invention.

The resulting micelle-drug composition is suitable for sustained-release of the drug in vivo, thereby enhancing the therapeutic effect of the drug. Such effect may be maximized by controlling the molecular weights and the relative ratio of the hydrophilic and hydrophobic blocks.

### Summary of the Invention

Accordingly, it is an object of the present invention to provide an effective carrier of hydrophobic drugs which may be used in preparing a pharmaceutically effective drug composition.

In accordance with one aspect of the present invention, there is provided a polymeric micelle drug composition which comprises a polymeric micelle drug composition comprising: a micelle of a block copolymer having a hydrophilic component and a hydrophobic component; and at least one hydrophobic drug incorporated into the micelle; wherein the hydrophobic component is a biodegradable polymer selected from the group consisting of polylactide, polyglycolide, poly(lactide glycolide), and polycaprolactone; and the hydrophilic component is poly(alkylene oxide), as defined in claim 1. Preferred embodiments are given in the subclaims. The present invention furthermore provides a process as defined in claims 8 to 10.

### Brief Description of Drawings

Fig. 1 is the GPC(gel permeation chromatography) trace of the polylactide-poly(ethylene oxide)-polylactide triblock copolymer(EL-3L-1)(column:MT3-MT4(Waters, U.S.A.), flow rate :10 ml/min, eluent: tetrahydrofuran).
Fig. 2 is the GPC(gel permeation chromatography) trace of the poly(ethylene oxide)-polycaprolactone diblock copolymer (EC-2C-1)(column:Asahipak GS 520H, eluent: distilled water).
Fig. 3 shows the release profiles of paclitaxel and cyclosporin incorporated in the EL-3L-2 copolymer micelle.
Fig. 4 shows the anticancer activity of paclitaxel incorporated in the EL-2L-2 copolymer micelle.

### Detailed Description of the Invention

The drug delivery system of the present invention comprises block copolymer micelles made of biodegradable polymers, and when administered, it decomposes in vivo into non-toxic small molecules by simple hydrolysis or by the action of enzymes. Biodegradable block copolymer micelles having an average diameter of 10 to 40 nm, are particularl suitable for formulating an injection composition of hydrophobic drugs which are either insoluble or only slightly soluble in water.

The block copolymer micelle of the present invention may be prepared by combining a biodegradable hydrophobic polymer selected among polylactide(PLA), polycaprolactone(PCL), poly(lactide glycolide) (PLGA ) and polyglycolide (PGA) with a hydrophilic polymer selected among poly(alkylene oxide). A hydrophobic drug may be delivered to a patient much more effectively when it is carried by the block copolymer micelle of the present invention and the sustained release of the drug stored in the micelle enhances the therapeutic effect of the drug.

The block copolymer used in the drug composition of the present invention may be a polymer of formula (I) or (II):

R₁-(-OCH₂CH₂-)ₘ-X (I)

X-(-OCH₂CH₂-)ₘ-X (II)

wherein,
R₁ is hydrogen or C₁₋₂₀ alkyl, preferably it is C₁₋₅ alkyl;
m is an integer larger than 2, preferably from 10 to 3,000; and
X is a biodegradable hydrophobic polymer segment having a molecular weight more than 100, preferably 300-100,000, and is selected from the group consisting of polylactide(PLA), polycaprolactone(PCL), poly(lactide glycolide)(PLGA) and polyglycolide (PGA).

The more preferable block copolymer which may be used in the drug composition of the present invention are di- or tri-block copolymers of formulae (III), (IV), (V) and (VI):

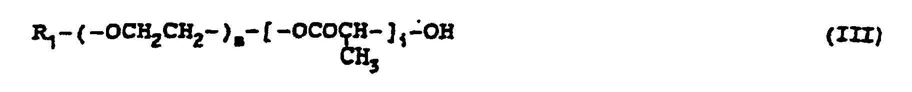

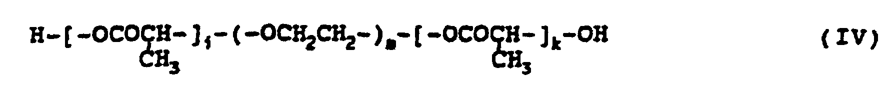

R₁-(-OCH₂CH₂-)ₘ-[-OCO(CH₂)₅-]ₗ-OH (V)

H-[-O(CH₂)₅CO-]ₙ-(-OCH₂CH₂-)ₘ-[-OCO(CH₂)₅-]ₚ-OH (VI)

wherein,
R₁ is hydrogen or C₁₋₂₀ alkyl, preferably it is C₁₋₅ alkyl;
i is an integer larger than 2, preferably from 2 to 1,500;
j and k are independently integers larger than 1, preferably from 2 to 1,000;
1 is an integer larger than 2, preferably from 2 to 700;
m is as described above; and
n and p are independently integers larger than 1, preferably from 2 to 500.

As described above, while poly(ethylene oxide) may be used as the preferred hydrophilic component of the block copolymer of the present invention, the hydrophobic component of the block copolymer of the present invention may comprise polylactide, polyglycolide, poly(lactide glycolide), polycaprolactone, derivatives thereof and the like having the following structures:

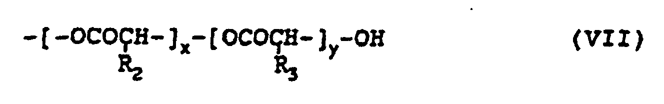

-[-OCO(CH₂)₅-]ₙ (VIII)

wherein,
R₂ and R₃ are independently H or CH₃;
x and y are independently integers larger than 2; and
n is an integer larger than 2, preferably from 2 to 500.

Diblock and triblock copolymers(AB type and ABA type) may be composed of a poly(ethylene oxide)(PEO) hydrophilic component(B) of and a polylactide(PLA) hydrophobic component(A), as shown in formulae (IX) and (X): 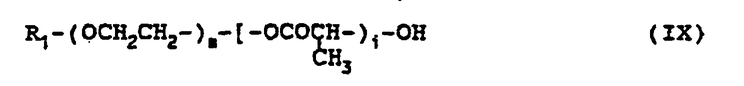 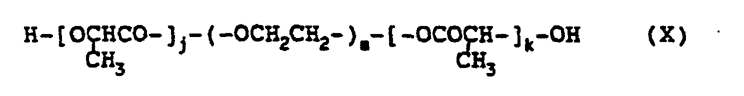 wherein,
i, j, k and m are as described above.

Diblock or triblock copolymers used in the present invention may be prepared by ring-opening polymerization. For example, the AB type diblock copolymer composed of PEO as the hydrophilic component(B) and PLA as the hydrophobic component(A) may be prepared by using PEO having a methoxy group at one terminal and a hydroxy group at the other terminal. The ABA type triblock copolymer may be prepared by using PEO having hydroxy groups at both terminals. The solubility of the micelle in water may be regulated by controlling the ratio of the hydrophilic component and hydrophobic component.

Suitable hydrophobic drugs which may be incorporated into the block copolymer micelle employed in accordance with the present invention are anti-cancer drugs such as paclitaxel, doxorubicin, teniposide, etoposide, daunomycin, methotrexate, mitomycin C and the like; antiphlogistic anodynes such as indomethacin, ibuprofen and the like; immunosuppressants such as cyclosporin and the like; hepatism remedies such as biphenyldimethylcarboxylate and the like; hormone compositions; antibiotics; chemotherapeutics; metabolic pharmaceuticals; digestive disease remedies; respiratory disease remedies; anti-allergic pharmaceuticals; central nervous system disease remedies; peripheral disease remedies; circulatory disease remedies; but not limited to those mentioned above.

In order to incorporate one or more drugs mentioned above into the block copolymer micelle, various methods described below may be used alone or in combination.

### (1) Stirring

A drug is added to an aqueous solution of a block copolymer, and stirred for 2 to 24 hours to obtain micelles containing the drug.

### (2) Heating

A drug and an aqueous solution of a block copolymer are mixed and stirred at 40 to 120 °C for 5 minutes to 24 hours and then cooled to room temperature while stirring to obtain micelles containing the drug.

### (3) Ultrasonic Treatment

A mixture of a drug and an aqueous solution of a block copolymer is subjected to an ultrasonic treatment for 1 second to 1 hour and then stirred at room temperature to obtain micelles containing the drug.

### (4) Solvent Evaporation

A drug is dissolved in an organic solvent and added to an aqueous solution of a block copolymer. Subsequently, the organic solvent is evaporated slowly while stirring, and then, filtered to remove non-solubilized drug.

### (5) Dialysis

A block copolymer is added to an organic solution of a drug and the mixture is dialyzed against a buffer solution and then water.

In the solvent evaporation or the dialysis method, suitable organic solvents for dissolving drugs are dimethylformamide(DMF), dimethylsulfoxide(DMSO), dioxane, chloroform, n-hexane, toluene, dichloromethane, ethyl acetate and the like.

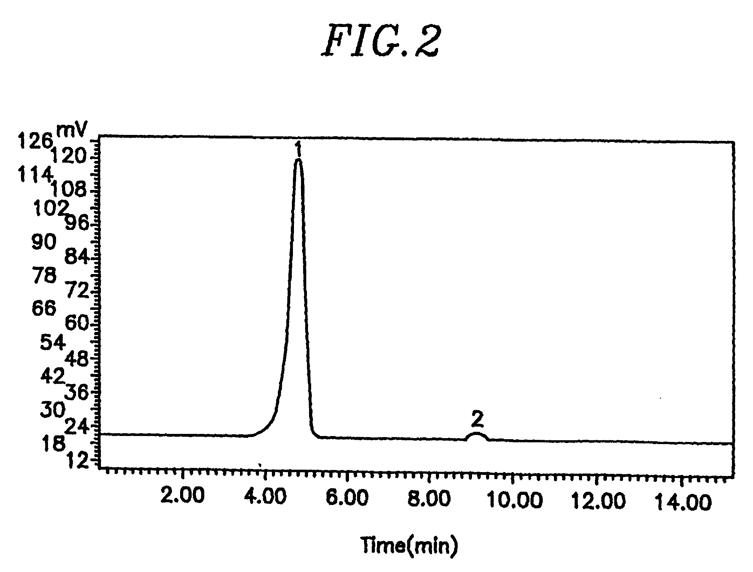

The block copolymers used in the present invention form stable micelles having an average size of 10-60 nm as shown in Table 1 of the Examples. Micelles of this size range are suitable for injection formulations and can avoid RES uptake while exerting EPR effect. The stability of the micelles is excellent, as can be seen from the gel permeation chromatography shown in Figure 2.

Further, a hydrophobic drug may be incorporated into the block copolymer micelle employed in according with the present invention by methods other than those described above, wherein the amount and physical state of the incorporated drug may vary depending on the composition of the block copolymer and also on the method of preparing the polymer micelle(Table 1). As the drug held in the compact core of the hydrophobic component is released in vivo in a controlled manner, the composition of the present invention is partially suitable for formulating drugs which are not amenable to conventional formulating techniques.

For example, paclitaxel is an outstanding anti-cancer agent but formulation thereof is difficult, mainly due to its low water-solubility. For this reason, a paclitaxel formulation containing Cremophor EL as the adjuvant is currently on the market, although Cremophor EL may cause some serious side effects. This particular formulation has other problems: i.e., it tends to form minute precipitates which require the use of a filter in the injection line; and the required period of administration is long, about 24 hours.

In contrast,the block copolymer micelle used in the present invention greatly enhances the solubility of paclitaxel, and the micelle-paclitaxel composition thus obtained is essentially non-toxic and exhibits enhanced anti-cancer therapeutic activity. As shown in Table 2, the amount of paclitaxel incorporated into the particular block copolymer micelles was 25.16 ± 3.27 %, while that of cyclosporin was 23.13 ± 2.31 %(Table 3). Further, the micelle-paclitaxel composition of the present invention released 85 % of the incorporated paclitaxel continuously over a period of 48 hours, while effectively preventing the cancer cells from growing. In case of the micelle-cyclosporin immunosuppressant composition, 40 % of the active ingredient was released continuously over a period of 48 hours.

The biodegradable diblock or triblock copolymer used in the present invention can form stable micelles which can incorporate hydrophobic drugs therewithin. The present invention thus provides a micelle-drug composition which is therapeutically more effective, and toxicologically much safer, than conventional formulations of hydrophobic drugs.

The following Preparation Examples and Examples are provided for purposes of illustrating certain aspects of the present invention only; they are not to be construed as limiting the scope of the present invention in any way.

### Preparation Example 1: Synthesis of Polylactide-Poly(ethylene oxide)-Polylactide Triblock Copolymer (EL-3L-0)

2 g of poly(ethylene glycol)(Mw 3400) was dried under a reduced pressure at 120 °C for 2 hours and 0.59 mg of stannous octoate(amount corresponding to 0.1% of D,L-lactide) was added thereto as a catalyst. The resulting mixture was subjected to a reduced pressure at 100 °C for 20 to 30 minutes to remove volatile compounds, mixed with 0.5882 g of D,L-lactide, and the mixture was reacted at 130 °C for 13 hours.

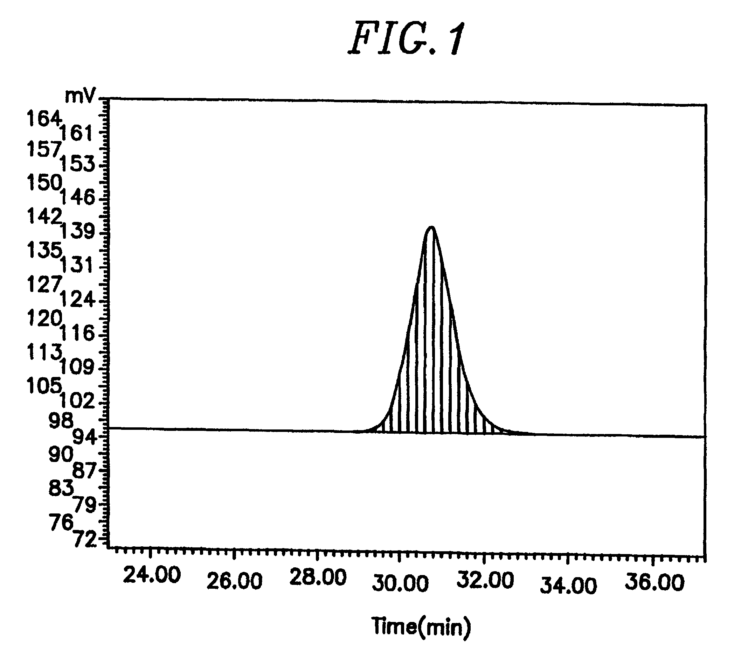

The block copolymer thus obtained was dissolved in 10 ml of chloroform and then an excess amount of diethyl ether was added with stirring to induce precipitation of the polymer. The precipitate was filtered and washed several times with diethyl ether, and then dried under a reduced pressure at 30 °C for one day to obtain 2.46 g of a triblock copolymer, polylactide-poly(ethylene oxide)-polylactide(PLA-PEO-PLA), designated EL-3L-0(yield 93%). The properties of this block copolymer are listed in Table 1 and the results of gel permeation chromatography are shown in Fig. 1.

### Preparation Example 2: Synthesis of Polylactide-Poly(ethylene oxide)-Polylactide Triblock Copolymer (EL-3L-1)

The procedure of Preparation Example 1 was repeated, except for using 2 g of poly(ethylene glycol)(Mw 3400) and 1.18 g of D,L-lactide, to obtain 2.95 g of a triblock copolymer, polylactide-poly(ethylene oxide)-polylactdie(PLA-PEO-PLA), designated EL-3L-1(yield 93%). The properties of this block copolymer are listed in Table 1.

### Preparation Example 3: Synthesis of Polylactide-Poly(ethylene oxide)-Polylactide Triblock Copolymer (EL-3L-2)

The procedure of Preparation Example 1 was repeated, except for using 2 g of poly(ethylene glycol)(Mw 3400) and 1.76 g of D,L-lactide, to obtain 3.46 g of a triblock copolymer, polylactide-poly(ethylene oxide)-polylactdie(PLA-PEO-PLA), designated EL-3L-2(yield 93%). The properties of this block copolymer are listed in Table 1.

### Preparation Example 4: Synthesis of Polylactide-Poly(ethylene oxide)-Polylactide Triblock Copolymer (EL-3L-3)

The procedure of Preparation Example 1 was repeated, except for using 2 g of poly(ethylene glycol)(Mw 3400) and 2.35 g of D,L-lactide, to obtain 3.87 g of a triblock copolymer of polylactide-poly(ethylene oxide)-polylactdie (PLA-PEO-PLA), designated EL-3L-3(yield 89%). The properties of this block copolymer are listed in Table 1.

### Preparation Example 5: Synthesis of Poly(ethylene oxide)-Polylactide Diblock Copolymer (EL-2L-0)

The procedure of Preparation Example 1 was repeated, except for using 2 g of monomethoxy poly(ethylene glycol)(Mw 2000) and 0.5 g of D,L-lactide, to obtain 2.28 g of a diblock copolymer of poly(ethylene oxide)-polylactdie(PEO-PLA), designated EL-2L-0(yield 91%). The properties of this block copolymer are listed in Table 1.

### Preparation Example 6: Synthesis of Poly(ethylene oxide)-Polylactide Diblock Copolymer (EL-2L-1)

The procedure of Preparation Example 1 was repeated, except for using 2 g of monomethoxy poly(ethylene glycol)(Mw 2000) and 1.0 g of D,L-lactide, to obtain 2.70 g of a diblock copolymer of poly(ethylene oxide)-polylactdie(PEO-PLA), designated EL-2L-1(yield 90%). The properties of this block copolymer are listed in Table 1.

### Preparation Example 7: Synthesis of Poly(ethylene oxide)-Polylactide Diblock Copolymer (EL-2L-2)

The procedure of Preparation Example 1 was repeated, except for using 2 g of monomethoxy poly(ethylene glycol)(Mw 2000) and 1.5 g of D,L-lactide, to obtain 3.22 g of a diblock copolymer of poly(ethylene oxide)-polylactdie(PEO-PLA), designated EL-2L-1(yield 92%). The properties of this block copolymer are listed in Table 1.

### Preparation Example 8: Synthesis of Polycaprolactone-Poly(ethyleneoxide)-Polycaprolactone Triblock Copolymer (EC-3C-1)

The procedure of Preparation Example 1 was repeated, except for using 2 g of poly(ethylene glycol)(Mw 3400) and 1.1765 g of caprolactone, to obtain 2.86 g of a triblock copolymer of polycaprolactone-poly(ethylene oxide)-polycaprolactone(PCL-PEO-PCL), designated EC-3C-1 (yield 90%). The properties of this block copolymer are listed in Table 1.

### Preparation Example 9: Synthesis of Poly(ethylene oxide)-Polycaprolactone Diblock Copolymer (EC-2C-1)

The procedure of Preparation Example 1 was repeated, except for using 2 g of monomethoxy poly(ethylene glycol)(Mw 2000) and 1.5 g of caprolactone, to obtain 3.2 g of a diblock copolymer of poly(ethylene oxide)-polycaprolactone(PEO-PCL), designated EC-2C-1(yield 91%). The properties of this obtained block copolymer are listed in Table 1.

### Preparation Example 10: Preparation of Polymeric Micelle

Each of the block copolymers synthesized in Preparation Example 1-9 was dissolved in distilled water or 0.1 M phosphate buffer(pH 7.4) to a concentration of 0.01 to 5 %(w/v) to obtain a polymeric micelle solution. The size of the micelle in each polymeric micelle solution measured by dynamic light scattering method was in the range from 10 to 60 nm as shown in Table 1. Polymeric micelle of this size is suitable for use as a drug carrier. The formation of the polymeric micelle was confirmed by the gel permeation chromatography in Fig. 2.

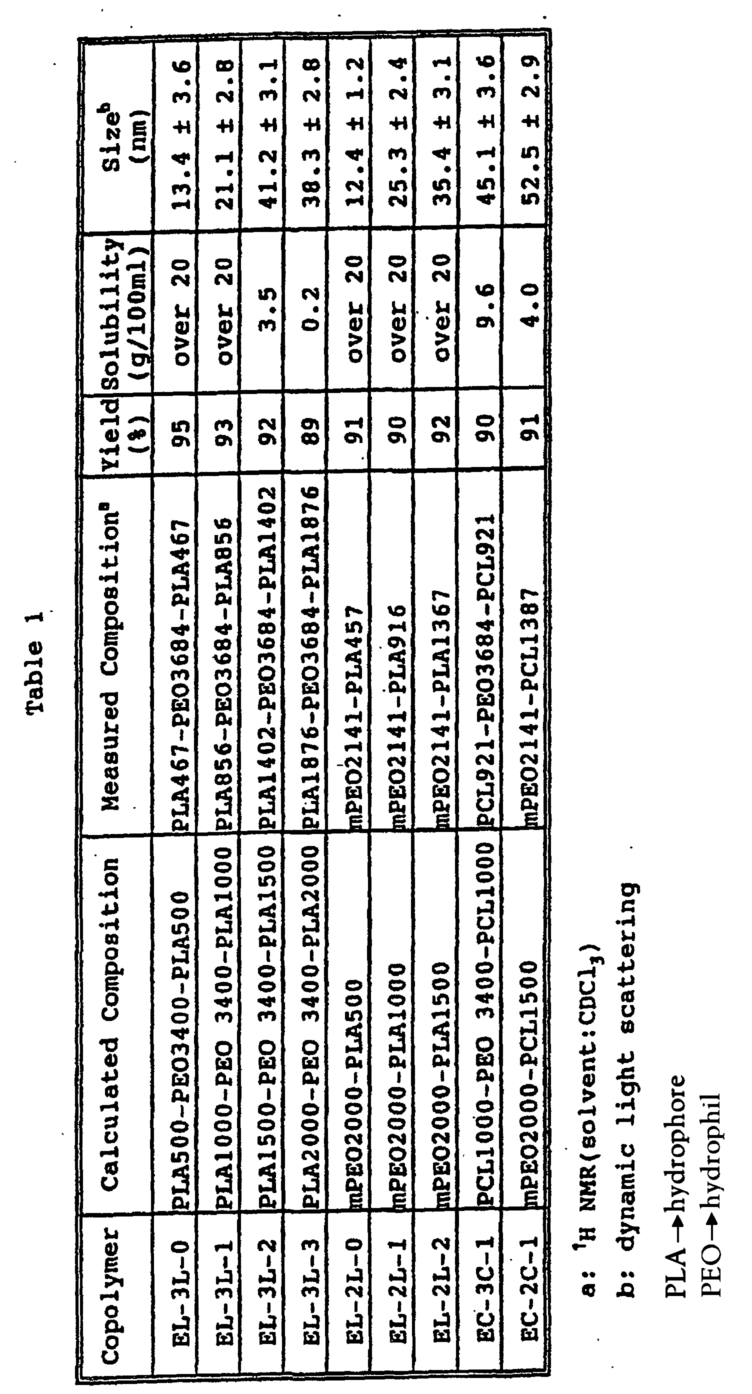

### Example 1: Preparation of Block Copolymer Micelle Containing Paclitaxel

### (1) Incorporation of paclitaxel into EL-3L-2, EL-2L-2 and EC-3C-1 by the stirring method

10 mg of each of the block copolymer EL-3L-2, EL-2L-2 and EC-3C-1 synthesized in Preparation Example 3, 7 and 8 was dissolved in 3 ml of distilled water and 5 mg of paclitaxel, an anticancer drug which is hardly-soluble in water, was added thereto and stirred for 2 hours. The resulting solution was filtered with a 0.45 µm membrane filter to remove unsolubilized paclitaxel and a clear solution of block copolymer micelles containing paclitaxel was obtained. The amount of paclitaxel incorporated into the polymeric micelle was determined by HPLC(column:Curosil-PFP(4.6*250 mm, 5 µm particle . size, Phenomenex, U.S.A.), mobile phase: acetonitrile/distilled water=45:55%(v/v)). The results are shown in Table 2.

### (2) Incorporation by Solvent Evaporation

EL-3L-2 synthesized in Preparation Example 3 was dissolved in distilled water, and a chloroform solution containing 3 mg of paclitaxel is slowly added thereto. The resulting mixture was stirred at room temperature overnight while allowing chloroform to evaporate. The resulting solution was filtered with a 0.45 µm membrane filter to remove unsolubilized paclitaxel and a clear solution of block copolymer micelles containing paclitaxel was obtained. This procedure was repeated using EL-2L-2 and EC-3C-1 synthesized in Preparation Example 7 and 8. The results are shown in Table 2.

### (3) Incorporation by Dialysis

5 mg of paclitaxel was dissolved in 5 ml of DMF. EL-3L-2 synthesized in Preparation Example 3 was added to the resulting solution and the mixture was stirred overnight. The mixture was dialyzed against 0.1 M phosphate buffer(pH 7.4) for 5 hours using a dialysis membrane(MWCO: 12000), and then against distilled water for 5 hours. The dialyzed solution was filtered with a 0.45 µm membrane filter and a clear solution of block copolymer micelles containing paclitaxel was obtained. This procedure was repeated using EL-2L-2 and EC-3C-1 synthesized in Preparation Example 7 and 8. The results are shown in Table 2.

These experiments shows that paclitaxel can be readily incorporated into the inventive polymeric micelles in an amount of upto 25.16 ± 3.23 %.

**Table 2**

| Copolymer | Paclitaxel Incorporation Ratio(%) | | |
|---|---|---|---|
| | Stirring | Solvent Evaporation | Dialysis |
| EL-3L-2 | 4.58 ± 0.36 | 15.53 ± 1.97 | 10.89 ± 1.57 |
| EL-2L-2 | 5.25 ± 0.46 | 18.44 ± 2.18 | 14.14 ± 1.94 |
| EC-3C-1 | 2.13 ± 0.22 | 25.16 ± 3.23 | 13.05 ± 1.63 |

### Example 2 : Preparation of Block Copolymer Micelle Containing Cyclosporin

### (1) Incorporation by Solvent Evaporation

10 mg of cyclosporin A, an immunosuppressant which is hardly-soluble in water, was dissolved in 1 ml of N,N-dimethyl acetamide and added slowly to a solution containing 20 mg of EL-3L-2 in 20 ml of distilled water. The resulting mixture was stirred overnight at room temperature while allowing N,N-dimethyl acetamide to evaporate off and the resulting solution was filtered with a 0.45 µm membrane filter to obtain a clear solution of the block copolymer micelles containing cyclosporin. This procedure was repeated using EC-2C-1 synthesized in Preparation Example 9. The results are shown in Table 3.

### (2) Incorporation by Dialysis

10 mg of cyclosporin A was dissolved in 5 ml of DMF. 20 mg of EL-3L-2 synthesized in Preparation Example 3 was added to the resulting solution and the mixture was stirred overnight. The mixture was dialyzed against 0.1 M phosphate buffer(pH 7.4) for 5 hours using dialysis membrane(MWCO: 12000), and then against distilled water for 5 hours. The dialyzed solution was filtered with a 0.45 µm membrane filter and a clear solution of block copolymer micelles containing paclitaxel was obtained. This procedure was repeated using EC-2C-1 synthesized in Preparation Example 9. The results are shown in Table 3.

These experiments shows that cyclosporin can be readily incorporated in the inventive polymeric micelles in an amount of upto 23.13 ± 2.31 %.

**Table 3**

| Copolymer | Cyclosporin Incorporation Ratio(%) | |
|---|---|---|
| | Solvent Evaporation | Dialysis |
| EL-3L-2 | 17.76 ± 1.97 | 14.96 ± 1.67 |
| EC-2C-1 | 23.13 ± 2.31 | 17.03 ± 1.84 |

### Example 3: Release Test

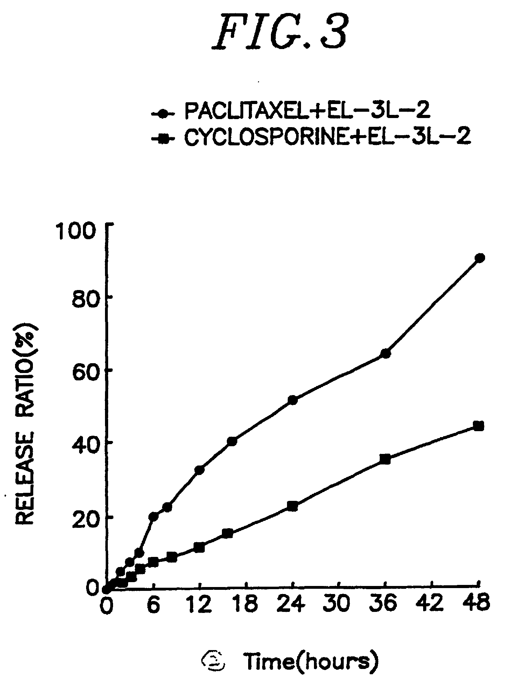

5 mℓ each of the paclitaxel- and the cyclosporin-containing EL-3L-2 copolymer micelle solution prepared in Examples 1 and 2, was placed in a dialysis sack(MWCO: 12,000). The sack was put into 1 ℓ of H₂O, and the amount of paclitaxel or cyclosporin released from the micelles was determined relative to the time. As can be seen from Fig. 3, the incorporated drugs show sustained release profiles.

### Example 4: Toxicity and Efficacy Test

10⁶ P388 leukemia cells were injected intraperitoneally to each member of three groups of mice, each consisting of six female BDF1 mice weighing 22 to 25 g.

24 Hours after the administration of leukemia cells, each of the mice in Group I was injected intraperitoneally with a vehicle(5% DMSO and 5% Cremophor saline solution) in an amount of 12.5 mg/kg, four times at a 24-hour interval, and each of the mice in Group II, was treated similarly with paclitaxel and the vehicle(5% DMSO and 5% Cremophor saline solution) under the same conditions.

On the other hand, each of the mice in Group III was administered intraperitoneally with 25 mg/kg of paclitaxel-containing EL-2L-2 copolymer micelle solution prepared in Example 1 (2), twice at 24 and 72 hours after the administration of the leukemia cells,.

The average survival time and the weight change in day 5 are listed in Table 4.

**Table 4**

| Group | Administered solution | Average survival time (hours) | Weight change(g) (at day 5) |
|---|---|---|---|
| I | Vehicle | 248±54 | -4.9 |
| II | Paclitaxel+vehicle | 407±81 | -13.9 |
| III | Paclitaxel+EL-2L-2 | 520±94 | -7.5 |

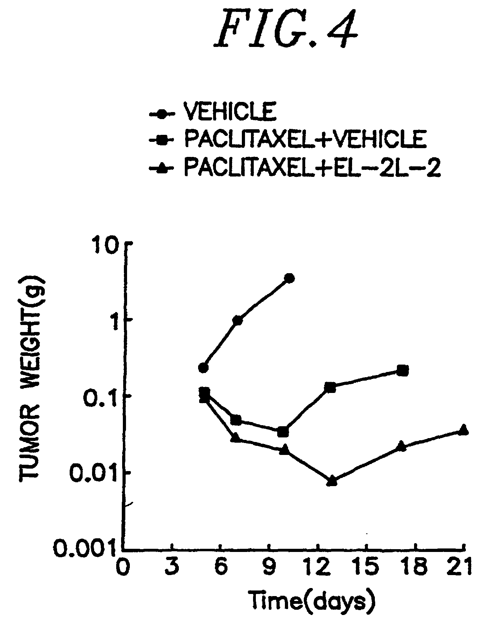

The anticancer activity of the paclitaxel-containing EL-2L-2 copolymer micelle was determined by measuring the tumor weights of the Group III mice relative to those of Groups I and II at a predetermined time. The result in Fig. 4 shows that the growth of tumor was efficiently inhibited by the polymeric micelle drug composition of the present invention.

As shown above, water-insoluble, hydrophobic drugs may be readily loaded into the biodegradable block copolymer micelles used in the present invention having a hydrophilic component and a hydrophobic component by way of either stirring, heating, ultrasonic treatment, solvent evaporation, dialysis and the like. The polymeric micelle drug composition thus obtained has a greatly improved pharmaceutical efficacy because an increased amount of the drug may be transferred effectively in patient's body.

## Claims

1. A polymeric micelle-type drug composition carrying a hydrophobic drug, comprising a polymeric micelle drug carrier composed of a block copolymer having a hydrophilic poly(alkylene oxide) component and a hydrophobic biodegradable component, and a hydrophobic drug physically entrapped within said polymeric micelle drug carrier,
wherein the block copolymer is not crosslinked and wherein the hydrophobic component is a biodegradable polymer selected from the group consisting of poly(lactic acid), poly(glycolic acid), poly(lactic-co-glycolic acid), and poly(ε-caprolactone).

2. The polymeric micelle-type drug composition of claim 1, wherein the hydrophilic component of the block copolymer is poly(ethylene oxide).

3. The polymeric micelle-type drug composition of claim 1, wherein the hydrophobic component of the block copolymer is poly(lactic acid).

4. The polymeric micelle-type drug composition of claim 1, wherein the block copolymer is a di-block or tri-block copolymer.

5. The polymeric micelle-type drug composition of claim 1, wherein the hydrophobic drug is paclitaxel, the hydrophilic component is poly(ethylene oxide) having a weight-average molecular weight of 1,000 to 6,000 daltons, and the hydrophobic component is poly(lactic acid) having a weight-average molecular weight of 1,000 to 8,000 daltons.

6. The polymeric micelle-type drug composition of claim 1, wherein the hydrophobic drug is paclitaxel, cyclosporin A or adriamycin.

7. The polymeric micelle-type composition of claim 1, wherein the weight average molecular weight ratio of the hydrophilic component and hydrophobic component of the block copolymer ranges from 1:0.5 to 1:3.

8. A process for incorporating a hydrophobic drug into a block copolymer micelle, which comprises the steps of:
preparing a micelle solution of a block copolymer having a hydrophobic component and a hydrophilic component, the hydrophobic component being a biodegradable hydrophobic polymer selected from the group consisting of poly(lactic acid), poly(glycolic acid), poly(lactic-co-glycolic acid) and poly(ε-caprolactone) and the hydrophilic component being poly(alkylene oxide); and
mixing the hydrophobic drug with the block copolymer solution and subjecting the mixture to stirring, heating, ultrasonic treatment, solvent evaporation or dialysis.

9. The process of claim 8, wherein the weight-average molecular weights of the hydrophilic and hydrophobic components of the block copolymer range from 1,000 to 6,000 daltons and from 1,000 to 8,000 daltons, respectively.

10. The process of claim 8, wherein the hydrophobic drug is paclitaxel, cyclosporin A or adriamycin.

## Patentansprüche

1. Polymere Arzneimittelzusammensetzung vom Mizellentyp, die ein hydrophobes Arzneimittel trägt, umfassend einen polymeren Mizellenarzneimittelträger, zusammengesetzt aus einem Blockcopolymer, mit einer hydrophilen Polyalkylenoxidkomponente und einer hydrophoben, bioabbaubaren Komponente, und einem hydrophoben Arzneimittel, physikalisch eingeschlossen in besagtem polymeren Mizellenarzneimittelträger, wobei das Blockcopolymer nicht vernetzt ist und wobei die hydrophobe Komponente ein bioabbaubares Polymer ist, ausgewählt ist aus der Gruppe bestehend aus Polymilchsäure, Polyglycolsäure, Polymilcnsäure-co-glycolsäure und Poly-ε-caprolacton.

2. Polymere Arzneimittelzusammensetzung vom Mizellentyp nach Anspruch 1, wobei die hydrophile Komponente des Blockcopolymeren Polyethylenoxid ist.

3. Polymere Arzneimittelzusammensetzung vom Mizellentyp nach Anspruch 1, wobei die hydrophobe Komponente des Blockcopolymeren Polymilchsäure ist.

4. Polymere Arzneimittelzusammensetzung vom Mizellentyp nach Anspruch 1, wobei das Blockcopolymer ein Diblock- oder Triblockcopolymer ist.

5. Polymere Arzneimittelzusammensetzung vom Mizellentyp nach Anspruch 1, wobei das hydrophobe Arzneimittel Paclitaxel ist, die hydrophile Komponente Polyethylenoxid mit einem Gewichtsmittel des Molekulargewichts von 1000 bis 6000 Dalton ist und die hydrophobe Komponente Polymilchsäure mit einem Gewichtsmittel des Molekulargewichts von 1000 bis 8000 Dalton ist.

6. Polymere Arzneimittelzusammensetzung vom Mizellentyp nach Anspruch 1, wobei das hydrophobe Arzniemittel Paclitaxel, Cyclosporin A oder Adriamycin ist.

7. Polymere Arzneimittelzusammensetzung vom Mizellentyp nach Anspruch 1, wobei das Verhältnis des Gewichtsmittels des Molekulargewichts der hydrophilen Komponente und der hydrophoben Komponente im Blockcopolymer von 1:0,5 bis 1:0,3 beträgt.

8. Verfahren zur Inkorporierung eines hydrophoben Arzneimittels in eine Blockcopolymermizelle, umfassend die folgenden Stufen:
Herstellen einer Mizellenlösung eines Blockcopolymeren mit einer hydrophoben Komponente und einer hydrophilen Komponente, wobei die hydrophobe Komponente ein bioabbaubares hydrophobes Polymer ist, ausgewählt aus der Gruppe bestehend aus Polymilchsäure, Polyglycolsäure, Polymilchsäure-co-glycolsäure, Poly-ε-caprolacton, und die hydrophile Komponente Polyalkylenoxid ist; und
Mischen des hydrophoben Arzneimittels mit der Blockcopolymerlösung und Unterwerfen der Mischung Rühren, Erwärmung, Ultraschallbehandlung, Lösungsmittelverdampfung oder Dialyse.

9. Verfahren nach Anspruch 8, wobei die Gewichtsmittel der Molekulargewichte der hydrophilen und hydrophoben Komponente des Blockcopolymeren von 1000 bis 6000 Dalton bzw. von 1000 bis 8000 Dalton betragen.

10. Verfahren nach Anspruch 8, wobei das hydrophobe Arzneimittel Paclitaxel, Cyclosporin A oder Adriamycin ist.

## Revendications

1. Composition de médicament de type à micelles polymères portant un médicament hydrophobe, comprenant un véhicule de médicament à micelles polymères composé d'un copolymère séquencé ayant un composant poly(oxyde d'alkylène) hydrophile et un composant biodégradable hydrophobe, ainsi qu'un médicament hydrophobe physiquement piégé à l'intérieur dudit véhicule de médicament à micelles polymères,
dans laquelle le copolymère séquencé n'est pas réticulé et dans laquelle le composant hydrophobe est un polymère biodégradable choisi dans le groupe formé par les poly(acide lactique), poly(acide glycolique), co-poly(acide lactique-acide glycolique) et la poly(ε-caprolactone).

2. Composition de médicament de type micelles polymères selon la revendication 1, dans laquelle le composant hydrophile du copolymère séquencé est un poly(oxyde d'éthylène).

3. Composition de médicament de type micelles polymères selon la revendication 1, dans laquelle le composant hydrophobe du copolymère séquencé est un poly(acide lactique).

4. Composition de médicament de type micelles polymères selon la revendication 1, dans laquelle le copolymère séquencé est un copolymère di-séquencé ou tri-séquencé.

5. Composition de médicament de type micelles polymères selon la revendication 1, dans laquelle le médicament hydrophobe est le paclitaxel, le composant hydrophile est un poly(oxyde d'éthylène) ayant une masse moléculaire moyenne en poids de 1 000 à 6 000 daltons, et le composant hydrophobe est un poly(acide lactique) ayant une masse moléculaire moyenne en poids de 1 000 à 8 000 daltons.

6. Composition de médicament de type micelles polymères selon la revendication 1, dans laquelle le médicament hydrophobe est le paclitaxel, la cyclosporine A ou l'adriamycine.

7. Composition de type micelles polymères selon la revendication 1, dans laquelle le rapport entre les masses moléculaires moyennes en poids du composant hydrophile et du composant hydrophobe du copolymère séquencé va de 1:0,5 à 1:3.

8. Procédé pour incorporer un médicament hydrophobe dans une micelle de copolymère séquencé, qui comprend les étapes consistant à :
- préparer une solution de micelles d'un copolymère séquencé ayant un composant hydrophobe et un composant hydrophile, le composant hydrophobe étant un polymère hydrophobe biodégradable choisi dans le groupe formé par les poly(acide lactique), poly(acide glycolique), co-poly(acide lactique-acide glycolique) et la poly(ε-caprolactone) et le composant hydrophile étant un poly(oxyde d'alkylène) ; et
- mélanger le médicament hydrophobe avec la solution de copolymère séquencé et à soumettre le mélange à une agitation, un chauffage, un traitement ultrasonique, une évaporation du solvant ou à une dialyse.

9. Procédé selon la revendication 8, dans lequel les masses moléculaires moyennes en poids des composants hydrophiles et hydrophobes du copolymère séquencé sont respectivement comprises entre 1 000 et 6 000 daltons et entre 1 000 et 8 000 daltons.

10. Procédé selon la revendication 8, dans lequel le médicament hydrophobe est le paclitaxel, la cyclosporine A ou l'adriamycine.
